# EUROPEAN PATENT APPLICATION

(11) **EP 4 718 458 A1**
(43) Date of publication of application: **01.04.2026**
(21) Application number: 24383048.6
(22) Date of filing: 30.09.2024
(51) Int. Cl.: G16B 15/20, G06N 10/00, G16B 40/20

(54) **METHOD AND SYSTEM FOR REDUCING A FOOTPRINT OF A PREDICTIVE COMPUTATIONAL MODEL, IN PARTICULAR FOR PREDICTING A STRUCTURE OF BIOLOGICAL PROTEIN STRUCTURES**

(71) Applicant: Multiverse Computing S.L., 20014 Donostia - San Sebastian (ES)
(72) Inventor: Jahromi, Saeed, 20014 Donostia / San Sebastián (ES); Orús, Román, 20014 Donostia / San Sebastián (ES)
(74) Representative: Sonnenberg Harrison Partners

(57) **Abstract**

The present invention proposes a system and method of reducing a footprint of a predictive computational model, and a method of predicting a protein structure using such a method of reducing footprint of the predictive model. The method of reducing a footprint of a predictive computational model comprises compressing the predictive computational model to come up with a compressed model, using a model compressor based on advanced network structures, and retraining the compressed model to adjust the compressed model and thereby produce an optimised predictive computational model with a reduced footprint.

## Description

### TECHNICAL FIELD

The present invention pertains to the intersection of artificial intelligence and quantum computing, specifically focusing on large language models for protein structure prediction. In particular, the present invention relates to a method and system for preparing a computational predictive model, in particular for reducing a footprint of a predictive computational model, and a method of predicting a structure of a biological protein using a predictive computational model, comprising a step of reducing a footprint of the predictive computational model, in particular for predicting a structure of biological protein structures.

### BACKGROUND

Protein structure prediction is a fundamental task in the field of bioinformatics, with wide-ranging applications in areas such as drug discovery and vaccine design. Traditional methods for protein structure prediction often rely on experimental techniques like X-ray crystallography and nuclear magnetic resonance (NMR) spectroscopy. However, these methods can be time-consuming, expensive, and sometimes even impossible for certain proteins.

With the advent of artificial intelligence (AI) and machine learning (ML), new computational methods have been developed to predict protein structures. Large language models (LLM), in particular, have shown promise in this area. However, these LLM models often require significant computational resources, including memory, which can limit their applicability and efficiency. This is particularly problematic when dealing with large datasets or complex protein structures.

Therefore, there is a need for more efficient computational methods, in particular for protein structure prediction that can overcome these limitations.

### SUMMARY OF THE INVENTION

To this end, the present invention proposes a method of reducing a footprint of a predictive computational model, comprising compressing, using a model compressor, the predictive computational model to produce a compressed model, wherein the predictive computational model comprises computational layers with data matrices. The compression comprises: identifying computational layers in the predictive computational model, reducing the data matrices in the identified computational layers via mathematical operations, wherein the data matrices are truncated to produce a compressed model, and re-training the compressed model to adjust parameters of the compressed model and thereby produce an optimised predictive computational model with a reduced footprint.

In an aspect, the predictive computational model has been pre-trained on training data and the compressed model is retrained with said training data. The training data may be protein structure datasets such as specific model variant.

In another aspect, the step of identifying computational layers comprises examination of the predictive computational model to determine a presence, sequence, and function of the computational layers in the predictive computational model, and reducing the dimensions of data matrices within the identified computational layers through a series of mathematical operations, resulting in a mathematical operator with a low parameter model.

The data matrices can be reduced through Singular Value Decompositions.

In an aspect, the reduction of the data matrices is iterative, with each iteration refining the approximation of the original data matrices, and determining of a Matrix Product Operator (MPO) with a low bond dimension.

In an aspect, the step of retraining of the compressed model includes running the compressed model through learning iterations with the training dataset, to output an updated compressed predictive computational model.

In an aspect, the method comprises adjusting the compressed predictive computational model after retraining, including adjusting parameters of the compressed predictive computational model.

The adjustment can be achieved by comparing predicted data by the compressed model against known actual data and modifying said parameters to reduce a difference between the predicted data and the actual data, in particular using backpropagation and gradient descent to improve the parameters of the compressed model.

The present invention also proposes a method of predicting a structure of a biological protein using a predictive computational model, the method comprising: loading the predictive computational model onto a computational hardware, which includes processing and storage units, applying the predictive computational model to output a first prediction, such as a first structure of biological protein, reducing a footprint of the predictive computational model by applying a method of reducing a footprint of a predictive computational model, comprising compressing the predictive computational model to come up with a compressed model, using a model compressor based on advanced network structures. The compression comprises identifying computational layers in the predictive computational model, reducing data matrices in the identified computational layers via mathematical operations, wherein the data matrices are truncated, to produce a compressed model, retraining the compressed model to adjust the compressed model and thereby produce an optimised predictive computational model that uses less storage, and configuring the system designed to execute a method for predicting the structure of biological protein structures.

In an aspect, the method of predicting a structure of a biological protein using a predictive computational model comprises pre-training the predictive computational model with a dataset and retraining the compressed model with the same dataset.

The compressed model can be adjusted by comparing a prediction of the compressed model with the first prediction.

The loading step can be carried out by executing commands or using an interface to transfer the predictive computational model, including model data and parameters into at least one storage unit of the computational hardware, to harness the computational power of the computational hardware to run the predictive computational model.

The invention also proposes a system configured to perform a method of any of the preceding claims.

The method proposes therefore loading a pre-trained predictive computational model onto the computational hardware. The predictive computational model is then applied to predict a structure of biological proteins, utilizing its pre-existing knowledge base and algorithms. The predictive computational model is compressed, reducing its memory footprint. This improves an overall efficiency.

The compression can identify computational layers within the predictive computational model and truncate data matrices to produce a more compact representation. After compression, a retraining with the original database of the compressed model is preformed to ensure the accuracy of the compressed model is maintained. This retraining process adapts the compressed model to its new, streamlined form. The result is an optimized compressed model that requires less storage space and is faster during learning and application phases.

With other words, the method proposes removing information from the large Language Model.

The predictive computational model's predictive performance remains high after compression, with improved precision and reduced power consumption. Therefore the compressed model is suitable for applications in chemistry and life sciences.

Following the initialization, the model is subjected to a compression technique utilizing a model compressor influenced by quantum-inspired tensor networks. This step is aimed at minimizing the model's memory requirements. It involves pinpointing and truncating weight matrices within the model's deep learning layers. The truncation process employs Singular Value Decompositions to transform the matrices into a Matrix Product Operator with a reduced bond dimension.

Subsequent to the compression, a retraining phase is done with an original database of protein structures. This retraining aims at maintaining the model's predictive accuracy post-compression. The retraining allows fine-tuning the model parameters to preserve or enhance the model's ability to predict protein structures accurately.

The result of these procedures is an optimized predictive computational model that utilizes less memory and operates faster during training and inference. This leads to improved precision, reduced processing time, and decreased power consumption, which are essential for the accurate prediction of protein molecular structures in applications such as drug and vaccine development.

### DESCRIPTION OF FIGURES

FIG. 1 illustrates, in a flowchart, operations for optimizing a predictive computational model for predicting biological molecular structures.
FIG. 2 illustrates, in a block diagram, a machine learning system.
FIG.3 is a flow chart of a compression step of a computational model.
FIG. 4 shows a compressor module.
FIG. 5 shows a system optimization and storage module.

### DETAILED DESCRIPTION

The invention will now be described on the basis of the drawings. It will be understood that the embodiments and aspects of the invention described herein are only examples and do not limit the protective scope of the claims in any way. The invention is defined by the claims and their equivalents. It will be understood that features of one aspect or embodiment of the invention can be combined with a feature of a different aspect or aspects and/or embodiments of the invention.

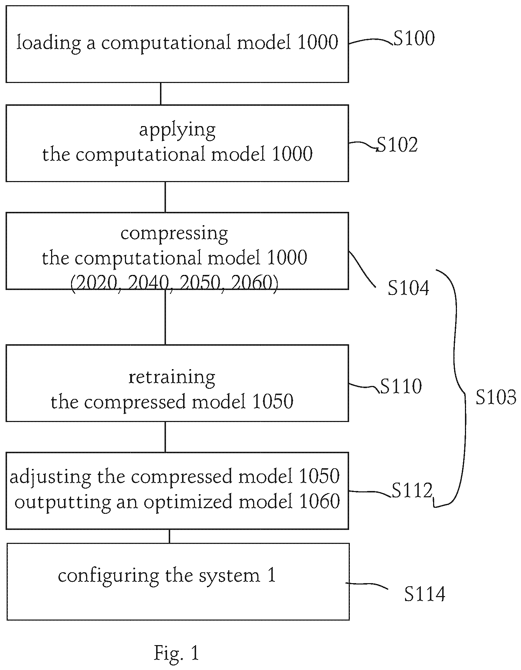

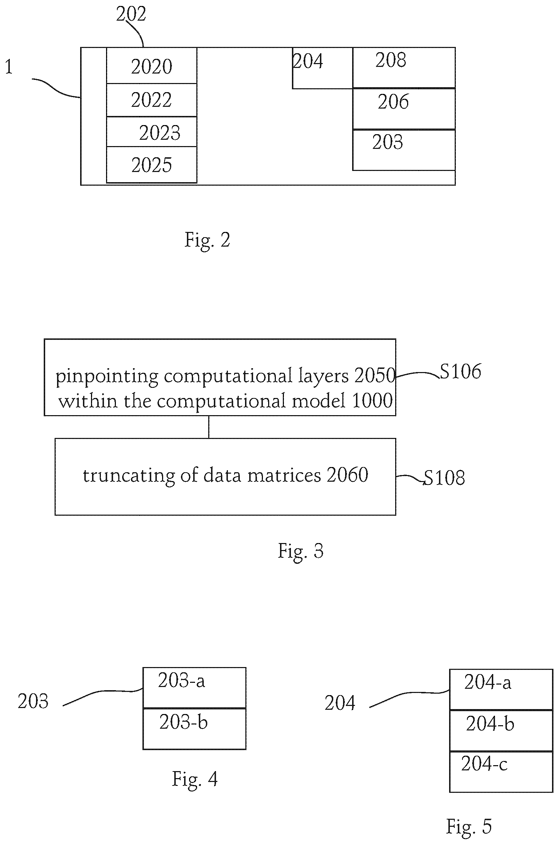

FIG. 1 illustrates, in a flowchart, a method for predicting biological molecular structures. As will become clear to the skilled person, the method for predicting biological molecular structures includes operations for preparing a predictive computational model, in particular reducing footprint of the predictive computational model, such as a predictive computational model for predicting biological molecular structures. The method of fig. 1 is described in conjunction with a machine learning system 1 as shown on FIG. 2.

The method includes loading a pre-trained advanced predictive computational model onto computational hardware, which includes processing and storage units, and applying the advanced predictive computational model to predict the structure of biological structures.

The method comprises a step 100 of transferring a predictive computational model 1000 that has been trained on protein structure datasets into a machine learning system 1 with a hardware module. With other words, a pre-trained advanced predictive computational model is loaded onto the computational hardware module 202.

This step 100 is an initialisation phase where the predictive computational model is placed into an operational state within a computational hardware 202.

In an aspect, the predictive computational model 1000 is tailored for the prediction of protein structures. The predictive computational model 1000 is trained to predict protein structures, such as ESM-Fold or its variants.

The predictive computational model 1000 can be pre-trained on a specific variant.

A System Configuration and Method Execution module 208 is provided for integrating and facilitating the functionality of the method, which utilizes quantum-inspired techniques to improve computational performance. The System Configuration and Method Execution module 208 includes hardware to execute to carry the method is carried out effectively, from loading the predictive computational model onto the hardware to the final production of an optimized model. The system configuration and method execution module 208 is thus essential for executing the method that enables quick and accurate protein structure predictions while minimizing the computational resources required.

The computational hardware 202 includes one or more processing units 2020, such as graphics processing units GPUs, which are designed to handle complex computations, and at least one storage unit 2022, which can store data and the predictive computational model 1000. A field programmable gate array (FGPA) 2023 for control logic that can also be connected to the processor CPU and one or more (GPU) and a field programmable gate array (FGPA) for control logic can also be connected to the one more processing units. The calculations can be spread around different ones of the GPUs.

The computational hardware 202 has computational power to manage complex calculations and large data sets.

The Advanced Computational Model and Hardware 202 can establish a computational environment, for example for analysing protein structures.

The transfer step S100 can be carried out by executing commands or using an interface 2025 to transfer the predictive computational model 1000 into the at least one storage unit 2022 of the computational hardware 202.

The predictive computational model comprises data 2020 and parameters 2040, including computational layers 2050 and data matrices 2060. Parameters are, for instance, the number of layers in the model, and the specifics of every layer, such as the number of neurons per layer, as well as possible truncation parameters of quantum-inspired tensor networks compressing weight matrices.

The aim of the transfer S100 of the predictive computational model 1000 onto the computational hardware 202 is to harness the computational power of the computational hardware 202 to run the predictive computational model. This is necessary due to the size and complexity of the predictive computational model 1000, which require substantial computational resources. The objective of this action is to set up the computational hardware 202 for subsequent processes that aim to enhance the performances of the predictive computational model 1000 in terms of memory usage, speed, and accuracy. For example, reduction of 40% to 60% with respect to the initial footprint can be achieved, yet no reduction of predictive capabilities.

After the transfer S100, the method comprises a step S102 of application of the predictive computational model 1000 to predict a structure of a biological protein. This step requires the predictive computational model 1000, which has been pre-trained on datasets of known protein structures, to process input data indicative of the biological proteins. The predictive computational model 1000 applies learned algorithms and parameters to the input data to generate a forecast of the structure of biological proteins.

In step S102, the predictive computational model 1000 operates within the computational environment 202, utilizing its pre-trained knowledge to interpret the input data. The process of predicting protein structures requires the predictive computational model 1000 to navigate through complex patterns that dictate how proteins fold in nature. The output of the predictive computational model output is a structure that can be evaluated for accuracy and relevance to the protein's function or potential as a target for therapeutic intervention.

In an aspect, the input data represent the sequence of amino acids of a target protein, and in an aspect, the structure of a biological protein is a protein molecular structure, such as a protein's three-dimensional conformation. In this aspect, the predictive computational model 1000's predictive function is applied to the task of determining the spatial arrangement of proteins. This involves computations that simulate protein folding by considering interactions between amino acids and the surrounding biological environment. The predicted protein structure can be utilized for further scientific analysis or practical applications such as drug design.

An identification module 206 is provided for the identification of biological structures, specifically protein molecular structures. The identification module 206 is responsible for the identification of the biological structures.

The method comprises a step S103 of reducing the footprint of the predictive computational model 1000. The step S103 aims at enhancing an ability to recognize and predict protein structures of the computing environment 1 with the predictive computational model 1000 through a compression technique based on quantum-inspired tensor networks, which reduces memory usage and increases the speed of learning and application.

Reducing the footprint of the predictive computational model 1000 comprises transforming the predictive computational model 1000 into a compressed model 1050. The compressed model 1050 is a more compact version that requires less memory for storage and operation.

More precisely, the method of reducing the footprint comprises the step S104 of compressing, by a model compressor 203, the predictive computational model 1000, to thereby reduce the size and complexity of the predictive computational model.

The aim of the compression step S104 is to enhance an overall operational efficiency of the predictive computational model 1000 by reducing its memory requirements. This is achieved without significantly impacting the predictive computational model's predictive performance. The result is a compressed model 1050, which has a reduced memory footprint compared to the predictive computational model 1000, whilst maintaining a high level of predictive accuracy.

Hence, the compressed model 1050 can be stored and processed with less memory, leading to faster processing times and reduced energy requirements during operation. This efficiency is particularly valuable in applications that involve large-scale data analysis, such as protein structure prediction, where the ability to quickly and accurately process information can greatly benefit research and development efforts in fields like chemistry and life sciences.

In an aspect, the model compressor 203 is configured to utilize network structures inspired by quantum tensor networks. The model compressor 203 operates by identifying redundancies within the predictive computational model 1000, in particular within the data structures and parameters, such as the number of layers, specifics of weights in weight matrices, number of neurons and connectivity, of the predictive computational model 1000, and by eliminating said redundancies, meaning irrelevant information in the mathematical implementation of the predictive model. The redundancies are removed in a manner that maintains the predictive computational model's ability to accurately predict protein structures.

In an aspect, the model compressor 203 is based on network structures that are quantum-inspired. The network structures are mathematical representations that allow for the efficient handling of the data structures and parameters of the predictive computational model 1000.

In one aspect, the predictive computational model 1000 is a pre-trained large language model, LLM. The pre-trained large language model is a large language model that specializes in protein structure prediction. The large language model is composed of multiple computational layers with data matrices, responsible for a different aspect of data processing. The identification of the computational layers is necessary to understand the sequence and function of each computational layer within the overall architecture of the predictive computational model.

The compression step S 104 is shown in Fig. 3. The compression step S104 comprises a step S106 of pinpointing computational layers 2050 within the predictive computational model 1000, followed by a step S 108 of truncating of data matrices 2060 to produce a more compact representation. The data matrices can be truncated through Singular Value Decompositions.

Data matrices 2060 of the predictive computational model 1000 can be reconfigured into a format that is more memory-efficient by truncating those correlations that are irrelevant for the mathematical description of the model. This is achieved by applying tensor networks, which are capable of representing complex, high-dimensional tensors as interconnected networks of simpler, lower-dimensional tensors.

The step S106 of pinpointing, i.e.. identifying and targeting, computational layers comprises examination of the predictive computational model, here a neural network, to determine the presence, sequence, and function of each of the computational layers. The examination is used for identifying which computational layers can be targeted for compression. The purpose of the step S106 of pinpointing specific computational layers is to enable selective compression of the predictive computational model 1000 to reduce its memory footprint while maintaining the integrity of its predictive capabilities.

Once the computational layers 2050 have been identified and targeted, in the next step S108, the method comprises reducing the dimensions of data matrices 2060 within the identified computational layers through a series of mathematical operations, resulting in a mathematical operator with a low parameter. The reduction step S108 is executed using Singular Value Decomposition (SVD), a linear algebra technique that decomposes a matrix into three matrices. The purpose of the reduction step is to retain significant components of the data matrices while discarding less significant information, thereby creating a compact representation of the predictive computational model.

In an aspect, the reduction of the data matrices is iterative, with each iteration refining the approximation of the original data matrices. The goal is to preserve valuable information while minimizing the overall parameter count. The outcome of this iterative process is the formation of a Matrix Product Operator (MPO) with a low bond dimension. The bond dimension in a tensor network representation limits the connections between tensors, which correspond to the layers of the predictive computational model 1000. A lower bond dimension indicates fewer parameters, leading to a more compact model.

The reduction of the data matrices involves the computational layers, the data matrices within the computational layers, and mathematical tools used for reduction (SVD).

With other words, the compression comprises an analytical phase where the structure of the predictive computational model is examined to gain an understanding necessary for the effective application of tensor network techniques for model compression, leading to an optimized model with improved performance metrics.

This allows creating a compressed model that is more efficient in terms of memory usage, computational speed, and power consumption.

The reduction of the size of the predictive computational model 1000 aims to balance accuracy with efficiency, so that resulting compressed model 1050 is more suitable for use in environments with limited memory resources. This balance between accuracy with efficiency allows for the application of the predictive computational model 1000 in various situations where computational efficiency is a significant consideration. It is possible to choose between the predictive computational model and the compressed model.

The compression step S104 can be performed by the compressor module 203, using the computational hardware 202. The compressor module 203 is shown in Fig. 4 and has a Model Compression and Network Structures module (203-a) and a Data Matrix Operations module (203-b) are critical for reducing memory usage and accelerating computations.

The Model Compression and Network Structures module (203-a) comprises a model compressor that utilizes quantum-inspired tensor networks to reduce the model's memory requirements. This process is essential for maintaining the model's predictive capabilities while optimizing its efficiency. a Data Matrix Operations module (203-b) is provided to truncate data matrices within the computational layers of the model. This is achieved through Singular Value Decompositions, which simplify the matrices into a Matrix Product Operator with a low bond dimension.

Reverting back to fig. 1, after the compression step S104, the method goes on with a step S 110 of updating the knowledge of the compressed model 1050. The updating can be made using a training dataset that contains information on protein structures, to adjust parameters of the compressed model 1050 to account for the reduced complexity due to the compression.

The updating step S110 is conducted after the predictive computational model 1000 has been compressed to ensure that it continues to predict protein structures accurately. The updating includes running the compressed model 1050 through learning iterations with the training dataset.

After compression, the system is retrained with an original database of protein structures to ensure the model's accuracy is maintained post-compression. This retraining adjusts the model to compensate for any loss in predictive capability due to the compression process. The outcome is an updated compressed predictive computational model which is optimized to require less storage, operates faster in both learning and application phases, and offers improved precision and energy efficiency.

This adjustment is achieved by comparing predicted data by the predictive computational model 1000 against known actual data and modifying said parameters to reduce the difference between the predicted data and the actual data. The parameters may include the number of layers, the number of neurons per layer, the number of tensors in the tensor network, and the truncation parameter of the tensor network (bond dimension) that truncates the correlations.

Techniques such as backpropagation and gradient descent may be used to systematically improve the parameters of the predictive computational model 1000 during this step, in order to optimize the different parameters of the model. Specific algorithms can be used, such as Adam optimizer.

In an aspect, the compressed model 1050 is updated using the original dataset it was initially trained on. This ensures that the performance of the compressed model 1050 remains aligned with the performance of the predictive computational model before compression, i.e. with the dataset's characteristics and the knowledge it represents, which is essential for maintaining the predictive computational model 1000 utility in applications such as drug and vaccine design.

The retraining process directly benefits the identification capabilities of the identification module 206. The result is a compressed model that identifies protein structures that operates more quickly, and consumes less power, yet without compromising the efficiency and precision.

It should be understood that having a compressed model is essential for applications where efficiency and resource management are vital.

In a final method step S112 an optimised compressed predictive computational model 1060 is produced that, after undergoing previous processes, exhibits enhanced characteristics in terms of memory usage, speed, precision, processing time, and power consumption.

The optimisation steps can be performed by a System Optimization and Storage module 204 shown on Fig.5. The module 204 is adapted to enhance the efficiency of a quantum-inspired large language model in protein structure prediction. The System Optimization and Storage module 204 can focus on optimizing storage, speed, and energy consumption, which are essential for applications in life sciences.

In an aspect, the System Optimization and Storage module 204 includes three sub-modules: a Model Precision Optimization sub-module 204-a, a Model Processing Time Optimization module 204-b; and a Model Power Consumption Optimization module 204-c.

The Model Precision Optimization sub-module 204-a is provided to ensure the accuracy of the predictive computational model or of the compressed model. The Model Processing Time Optimization module 204-b is provided to enhance the speed of the predictive computational model or of the compressed model.

The Model Power Consumption Optimization module 204-c is provided reduces energy usage of the predictive computational model or of the compressed model.

Together, the Model Precision Optimization sub-module 204-a, the Model Processing Time Optimization module 204-b, and the Model Power Consumption Optimization module 204-c contribute to a streamlined predictive model.

The updating module 204 is involved in the retraining of the compressed model 1050. In an aspect, the original database of protein structures is used a comprehensive input data. The retraining refines the compressed model's accuracy after compression, ensuring that the predictive capabilities are maintained.

In particular, the Model Precision Optimization sub-module 204-a enhances the accuracy of the model by fine-tuning the model parameters to match protein folding patterns, which can be done by ramping up accuracy with a small retraining phase in specific datasets. The Model Processing Time Optimization module 204-b accelerates the model's learning and application phases through algorithmic improvements and hardware utilization strategies, reducing the time required for training and inference the Model Power Consumption Optimization module 204-c optimizes the computational processes to reduce power consumption, which is beneficial for large-scale deployments where energy consumption is a significant consideration.

The updated compressed model 1060 is compact in storage, faster in processing, and energy-efficient, making it a robust tool for protein structure prediction.

The resulting optimised model demonstrates a reduction in memory requirements compared to its original form. This is a result of the compression techniques, particularly the truncation of data matrices in step 108, which allows the model to operate effectively even with limited hardware resources.

In addition, due to the reduced number of parameters from the compression process, the compressed model 1050, and the updated compressed model 1060, can perform training and inference in less time, which facilitates faster iterations in applications such as drug and vaccine development.

Another benefit of the method is the enhancement of the precision in predicting protein molecular structures. Indeed, the retraining step S 110 with the original database in ensures that the compressed model maintains its accuracy in structure prediction, which is essential for the development of effective pharmaceuticals.

Another benefit is an improvement of the processing time. This improvement is not only about the speed of the model's operations but also encompasses the efficient management of computational tasks.

Finally, the compressed model has an increased efficiency in power consumption. With the compressed model being less demanding on memory and operating at a faster rate, it also tends to require less energy, which is beneficial for computations on a large scale or in environments where energy resources are limited.

Together, these enhancements contribute to the development of an optimised predictive computational model that is better suited for tasks such as the prediction of protein structures.

The reduced memory footprint, increased speed, maintained precision, improved processing time, and reduced power consumption make the optimised model a more practical tool for applications in chemistry and life sciences.

Once the compressed model has been optimized, the method comprises a Step S 114 of configuring the system designed to execute a method for predicting the structure of biological protein structures. With other words, the configuration corresponds to the final integration of various hardware and software components to create a functional unit capable of executing the specified method.

The configuring of the system includes setting up computational hardware, which consists of processing units like GPUs and memory units, and ensuring that the software components, such as the pre-trained advanced predictive computational model and the model compressor, are properly integrated.

The actions taken during the configuration involve establishing the hardware to run the predictive computational model, integrating the model compressor to manage the model's memory requirements, and confirming that all layers and data matrices within the model are correctly identified and processed (Steps 106 and 108). The system must also be capable of undergoing a retraining process with the original dataset to maintain or enhance its predictive performance. The final optimized model is expected to demonstrate reduced memory usage, improved training and inference times, enhanced precision, and increased energy efficiency.

The objective of the configuration step 114 is to ensure that all these components function together to enable the prediction of protein molecular structures with the outlined benefits. This includes loading the specific pre-trained model variant and applying the model to predict protein structure.

In essence, this is an implementation phase where the system is assembled and adjusted to carry out the method of predicting protein structures using a large language model. The system and method are efficient in terms of memory and performance, ready for applications in fields such as drug and vaccine design.

The Quantum-Inspired Protein Structure Prediction System (200) addresses the challenge of high memory usage in AI-based protein structure prediction, enhancing accuracy, speed, and efficiency. The system comprises the Advanced Computational Model and Hardware (202), which includes a pre-trained model and computational hardware, essential for protein structure prediction. The system, incorporating a pre-trained model like ESM-Fold, is loaded onto machine learning hardware that includes GPUs and memory units.

The System Configuration and Method Execution component includes the system and method required to execute the protein structure prediction process. It ensures that the method is carried out effectively, from loading the predictive computational model onto the hardware to the final production of an optimized model.

In summary, the compressed predictive computational model, i.e. the compressed large language model, is retrained using the dataset of protein structures. This retraining is necessary to refine the predictive computational model 1000's ability to predict protein structures after undergoing compression, aiming to achieve accurate predictions while benefiting from reduced memory usage and enhanced efficiency during training and inference.

. In summary, the method is characterized by compressing the advanced predictive computational model using a model compressor based on advanced network structures, identifying computational layers in the advanced predictive computational model, truncating data matrices in these layers via mathematical operations, producing a mathematical operator with a low parameter, retraining the system with a data source, and producing an optimised predictive computational model that uses less storage, is faster in model learning and application, and allows for better precision, processing time, and power consumption in the prediction of biological molecular structures.

## Claims

1. Method of reducing a footprint of a predictive computational model, comprising compressing, using a model compressor, the predictive computational model to produce a compressed model, wherein the predictive computational model comprises computational layers with data matrices, and wherein the compression comprises:
identifying computational layers in the predictive computational model,
reducing the data matrices in the identified computational layers via mathematical operations, wherein the data matrices are truncated to produce a compressed model,
re-training the compressed model to adjust parameters of the compressed model and thereby produce an optimised predictive computational model with a reduced footprint.

2. The method of claim 1, where the predictive computational model has been pre-trained on training data and wherein the compressed model is retrained with said training data, in particular wherein the training data are protein structure datasets such as specific model variant.

3. The method of claim 1, wherein the step of identifying computational layers comprises examination of the predictive computational model to determine a presence, sequence, and function of the computational layers in the predictive computational model, and reducing the dimensions of data matrices within the identified computational layers through a series of mathematical operations, resulting in a mathematical operator with a low parameter model.

4. The method of the claim 1, wherein the data matrices are reduced through Singular Value Decompositions.

5. The method of claim 4, wherein the reduction of the data matrices is iterative, with each iteration refining the approximation of the original data matrices, and determining of a Matrix Product Operator (MPO) with a low bond dimension.

6. The method of claim 1, wherein the step of retraining of the compressed model includes running the compressed model 1050 through learning iterations with the training dataset, to output an updated compressed predictive computational model.

7. The method of claim 1, comprising adjusting the compressed predictive computational model after retraining, including adjusting parameters of the compressed predictive computational model.

8. The method of claim 7, wherein the adjustment is achieved by comparing predicted data by the compressed model 1050 against known actual data and modifying said parameters to reduce a difference between the predicted data and the actual data, in particular using backpropagation and gradient descent to improve the parameters of the compressed model.

9. A method of predicting a structure of a biological protein using a predictive computational model, the method comprising:
loading the predictive computational model onto a computational hardware, which includes processing and storage units,
applying the predictive computational model to output a first prediction, such as a first structure of biological protein,
reducing a footprint of the predictive computational model by applying a method of reducing a footprint of a predictive computational model, comprising
compressing the predictive computational model to come up with a compressed model, using a model compressor based on advanced network structures,
wherein the compression comprises
identifying computational layers in the predictive computational model, reducing data matrices in the identified computational layers via mathematical operations, wherein the data matrices are truncated, to produce a compressed model,
retraining the compressed model to adjust the compressed model and thereby produce an optimised predictive computational model that uses less storage, configuring the system designed to execute a method for predicting the structure of biological protein structures.

10. The method of claim 9, comprising pre-training the predictive computational model with a dataset and retraining the compressed model with the same dataset.

11. The method of claim 9 or 10, wherein the compressed model is adjusted by comparing a prediction of the compressed model with the first prediction.

12. The method of claim 9, comprising wherein the loading step is carried out by executing commands or using an interface to transfer the predictive computational model, including model data and parameters into at least one storage unit of the computational hardware, to harness the computational power of the computational hardware to run the predictive computational model.

13. A system configured to perform a method of any of the preceding claims.
